# EUROPEAN PATENT APPLICATION

(11) **EP 2 450 066 A1**
(43) Date of publication of application: **09.05.2012**
(21) Application number: 10360037.5
(22) Date of filing: 19.10.2010
(51) Int. Cl.: A61L 27/44, A61L 27/56

(54) **New hybrid implant**

(71) Applicant: PROTIP SAS, 67000 Strasbourg (FR); Université de Strasbourg, 67081 Strasbourg Cedex (FR); Institut National de la Santé et de la Recherche Médicale - Inserm, 75854 Paris Cedex 13 (FR)
(72) Inventor: Lavalle, Philippe, 67370 Wintzenheim (FR); Vrana, Engin, 67200 Strasbourg (FR); Vautier, Dominique, 67130 Wisches (FR)
(74) Representative: Barny, Luc

(57) **Abstract**

The present invention relates to a hybrid implant for the support and/or the replacement of tissue, a method for its preparation and its use. According to the invention, the hybrid implant is made of a polymeric scaffold combined with a mechanically stable template. It is particularly suitable for the disorders and pathologies where the support and/or the replacement of a tissue is necessary. The hybrid implant is particularly designed for cell colonization at a particular part of the implant or the whole implant, in order to restore the tissue functioning.

## Description

The present invention relates to a hybrid implant for the support and/or the replacement of tissue, a method for its preparation and its use. According to the invention, the hybrid implant is made of a polymeric scaffold combined with a mechanically stable template. It is particularly suitable for the disorders and pathologies where the support and/or the replacement of a tissue is necessary. The hybrid implant is particularly designed for cell colonization at a particular part of the implant or the whole implant, in order to restore the tissue functioning.

In animals, and especially in mammals, tissues are of utmost importance in performing various functions. In case of dysfunction, paralysis or loss of a tissue, there is a need of support and/or replacement of said dysfunctional, paralyzed or lost tissue. It is well known by one skilled in the art that from an anatomical point of view some tissues can be supported and/or replaced by biocompatible implants made of silicon, minerals, ceramics, metal, metal alloys or combinations thereof. But it is also well known that such implants, even if they restore partially or totally the mechanical function of the tissue, it is difficult to restore the tissue functioning and when this is the case, such implants have some disadvantages such as fast degradation in time, colonization by inflammatory cells, poor function restoration, poor mechanical properties and sometimes manufacturing problems.

The use of three-dimensional supports for cell growth is known from the prior art. Such support allows cell colonization and is used in vitro but also as an implant for in vivo application. For in vitro cell culture, the structure of the three-dimensional support has to be porous enough to allow the nutrient and waste flow. Moreover the average pore size of said structure must be large enough to permit the movement of the cells into the structure so that the colonization by the cells is as complete as possible according to the environmental conditions. In vivo the problem is different since there are a lot of different cell types able to colonize the implanted structure; some of them being desired cells and the other being undesired such as inflammatory cells, bacteria or fungi. A solution to this problem is the designing of a tissue engineering scaffold that allows a selection of the cell type able to colonize the structure or the structure part. The porosity of said tissue engineering scaffold determines the ability of the cells to move within the structure so it allows a cell type selection according to its size and properties. The control of the porosity of the scaffold allows in vitro culture of artificial tissues or cellular migration and growth within the whole body of a solid implant as an example. To date, artificial tissues are designed in vitro. The development of artificial tissues results first from various techniques such as freeze-drying, salt leaching, and rapid prototyping to build synthetic scaffolds or cell encapsulation within hydrogels. However, most of these methods have the drawback of producing isotropic pore structures. Therefore, such scaffolds are suitable only as far as one cell type is desired and the target area is relatively homogenous such as cartilage or bone.

Actually, tissues in physiological conditions exhibit functional gradient in order to fulfil their biological and mechanical requirements. Tissues are not composed of only one cell type but of several cell types with different and specific needs. Therefore, a tissue engineering scaffold should also reproduce the gradients across a spatial volume of the tissues (Leong et al., J Mech Behavior, 2008). Consequently a good tissue engineering scaffold should mimic said functional gradient and this renders its design and fabrication process very complex. In order to manufacture such tissue engineering scaffold, several methods where described and tested.

The use of a porous biocompatible gradient scaffold composed of polymers comprising at least one synthetic or natural polymer, ceramic, metal, extracellular matrix protein or an analogue thereof is described in WO 2006/034365 as an example. The manufacturing process is based on freeze-drying, and then exposition to a gradient of solutions containing cross-linking agents. The patent application WO 2003/022319 describes another tissue engineering scaffold made of a biocompatible polymer. The polymer can be collagen, elastin, or a mixture thereof. A preferred embodiment involves collagen, material that is intrinsically porous. The pores are formed during the moulding process and their diameter is not controlled. Several techniques allowing the manufacture of tissue engineering scaffolds from biodegradable and bioresorbable polymers have been described to date. With regards to synthetic polymers, such techniques are based on solvent casting-particulate leaching, phase separation, gas foaming and fibre meshes. With regards to natural polymers the techniques used are different. All the gradient scaffolds described above can be used for tissue function restoration but they will never insure the mechanical role of support because of the nature of their components.

To date, the use of porous three-dimensional bodies as implants for support and/or replacement of tissue in mammalians is well-known. Actually, such implants are made of biocompatible material such as ceramic, silicone, glass, metal or metal alloys. If the material in itself is not porous enough to allow the cellular colonization, it can be coated with an appropriate porous layer. A particularly efficient implant containing porous titanium or titanium alloy is described in EP0856299 (Debry et al); said implant being a phonatory implant. The parts of said implant made of microporous titanium are in contact with the mucous membranes of the implantation area. The size of pores of said implant can be optimized using the manufacturing process described in EP1940480 (Debry et al.). Such an implant made of titanium or of titanium alloy has a controlled, regular and open porosity, and more precisely a microporosity that allows and helps cell colonization. Such an implant is perfectly suitable for compensating the mechanical and some functional aspects of the tissue in need thereof. The disadvantage of said implant is that there is no gradient of porosity in order to restore full functionality of the tissues of the implanted area.

The combination of a mechanically stable template and a gradient of growth factors is also known (see US 11/614,665 Peckham) especially in the field of bone reconstruction. The mechanical support is ensured by a biocompatible and biodegradable solid material such as hydroxyapatite and a layer of growth factors is ensuring the cell fixation and development. The higher concentration of growth factor is dispersed in the portion of the implant where new bone tissue is needed and the lower concentration of growth factor is dispersed where new cartilage tissue is needed. Such a growth factor layer is not a tissue engineering scaffold but this principle results in an interesting effect of integration of the bone implant.

The combination of porous hydroxyapatite ceramic implant and a biodegradable polymer included in the pores of the implant is also known (US 12/621,274 Aizawa). In such a hybrid material for bone repair or replacement, the polymer layer is not porous and the hydroxyapatite ceramic implant has no controlled open porosity. The implant is to be positioned in a physiological area where bone reconstruction is needed. In fact, hydroxyapatite and natural bones have very different properties. Hydroxyapatite has a higher Young's modulus, a lower fracture toughness and it is brittle. The polymer layer is aimed at increasing the mechanical properties of the porous hydroxyapatite ceramic implant in order for those properties to be as similar as possible to natural bones.

All the materials and implants described above have disadvantages such as mechanical support with poor or no tissue function restoration, or function restoration with a weak mechanical support. Moreover, in addition to the mechanical and function restoration requirement, there is a need for implant colonization by the host tissue in order for the implant to be efficient and reliable. As described above, the use of biocompatible solid material in tissue support and/or replacement may lead to undesired stress around the junctions of that artificial material and the natural tissues. Such undesired stress will eventually lead to implant colonization by undesirable cells, bacteria and fungi, and finally to integration failure. Thus it remains a need for a new hybrid material that would have the benefits of solid biocompatible implants, but with mechanical and functional properties similar to those of natural tissues.

The present invention overcomes the drawbacks of prior art by providing the superposition of various porosities on a same implant, those porosities being chosen according to the tissue nature of the area where the implant is to be positioned. The hybrid implant according to the invention ensures the mechanical role of support and the restoration of the tissue function at the target area. The inventors have surprisingly discovered that the superposition of porosities, according to their positioning on the hybrid implant, are allowing or preventing the colonization of the implant by various cell types. The present invention relies on a gradient of porosity applied on a mechanically stable template with open porosity. Thus the hybrid implant according to the invention cumulates the mechanical and functional requirements of an implant for tissue support and/or replacement. Such a hybrid implant allows the control of the level of integration between the host tissue environment and the implant.

Other aspects and advantages of the hybrid implant according to the invention will be apparent from the following description and drawings.

The figures detailed below will illustrate in more details the present invention.
Fig. 1: a 3D model of a material with open porosity according to the prior art.
Fig. 2: a 3D model of a standard tissue engineering scaffold according to the prior art.
Fig. 3: a cross-sectional view of the tissue engineering scaffold showed on Figure 2.
Fig. 4: a general view of a standard microporous hybrid implant according to the invention.
Fig. 5: a 3D model of the implant according to the invention with polymer tissue engineering scaffold (1) that induces a pore gradient from the bottom to the top of said implant.
Fig. 6: a cross-sectional view of the hybrid microporous implant according to the invention. It displays a mechanically stable template (2) with regular beads in contact with each other and a pore gradient formed by a polymer tissue engineering scaffold (1) from the bottom to the top.
Fig. 7: detail of the porosity gradient obtained in the invention. The blanks due to the open porosity of the titanium implant are infused with a polymer material; the porosity of said polymer material decreases from bottom to top. An optional smooth or nanoporous film layer can be added.
Fig. 8: Details of the nanoporous top film layer
Fig. 9: a higher magnification of the nanoporous top film layer.
Figure 10: a 3D view of a tracheal implant made of microporous titanium according to the prior art.
Figure 11: a 3D view of a hybrid microporous tracheal implant according to the invention.
Figure 12: a 3D view of a hybrid microporous implant for tooth replacement.

The present invention relates to a hybrid microporous implant for the support and/or the replacement of tissue, comprising a polymer tissue engineering scaffold which porosity can be controlled, combined with a mechanically stable template with open porosity.

As stated above, controlling the porosity of the tissue engineering scaffold is of utmost importance since the types of cell that are able to colonize the implant or the implant part determine the level of integration of an implant. In a mammal, and especially in humans, some implantation areas are very complex and the fixation and growth of several types of cells have to be helped and favoured after an implant insertion. To increase the integration of the implant, the inventors have used a tissue engineering scaffold that has a porosity gradient. This allows some parts of the implant to be inaccessible or poorly accessible to the inflammatory cells and especially to fibroblasts, while some other parts are open to colonization by cells such as epithelial cells.

The open porosity of the mechanically stable template is also of utmost importance since the size of the pores and the regularity of the network they are part of is also very important for the cell fixation, movement and growth. The template can be made of any material that is not biodegradable or poorly biodegradable such as metal or metal alloys, ceramics, minerals, silicon or combinations thereof. To date some techniques allow a perfect control of the porosity of a template according to the invention for various materials: for hydroxyapatite (Liu, Biomaterials, 1996, Vol. 17, n°20 p. 1955-1957), for titanium (EP 1940480 Debry et al.), for glass (Vogel et al., CERAMICS, 2000, Vol. 44 n°1 p. 9-13), etc. The manufacture and use of mixed porous materials such as glass/metal or glass/ceramics have also been reported. Some of those materials are particularly suitable for some tissue support and/or replacement and the others will be more suitable for other tissues according to their physiological and mechanical properties.

The mechanically stable template can be made of synthetic or natural non-biodegradable polymers, ceramics, metals, metal alloys, glass, silicon and combinations thereof. On one hand, in order to perform its role of mechanical support, the template has to be poorly or non-biodegradable. On the other hand, in order to perform its role of metabolic and/or physiological tissue function restoring, the template needs to be perfectly biocompatible and porous in order to be completely colonized by cells. The porosity of the template needs to be adjusted to the environment area of its implantation site and in the meanwhile said porosity needs to be unfavourable to the colonization by inflammation cells and fibroblasts.

The mechanically stable templates are required to be biocompatible and poorly or not biodegradable. Adequate material for manufacturing such mechanically stable templates with open porosity is metal or metal alloy.

In the present invention, the use of a template made of microporous titanium is preferred since the technique described in EP 1940480 is very efficient in obtaining the required regular open porosity. Said technique is based on electrical discharge sintering of titanium microspheres in a selected mould. Titanium is a biocompatible and not biodegradable material. The method described in EP1940480 (Debry et al.) allows the manufacture of an appropriate template displaying open porosity and with regular pores of a determined size. Microporous titanium is made up of a three-dimensional juxtaposition of plain titanium beads (see standard NF ISO 5832-2). The titanium implant manufactured by that method has an open porosity characterized by interspheroid spaces having a dimension approximately equal to one third of the diameter of the powders used: with powders of 150 to 500 µm the interspheroid spaces will have a size between 50 and 150 µm. Consequently, the porosity of the mechanically stable template made of microporous titanium is easily controllable. The mechanical stability of the template and its controlled porosity allow a perfect adaptation to the environment, and especially to the natural cell types, of the area where the implant is to be inserted.

In one embodiment, the present invention relates to a hybrid microporous implant for the support and/or the replacement of tissue, comprising a polymer tissue engineering scaffold (1) which porosity can be controlled, combined with a mechanically stable template (2) with open porosity made of microporous titanium.

The present invention relates also to a hybrid microporous implant for the support and/or the replacement of tissue, characterized in that it comprises a polymer tissue engineering scaffold (1) with a gradient of porosity, combined with a mechanically stable template (2) with open porosity.

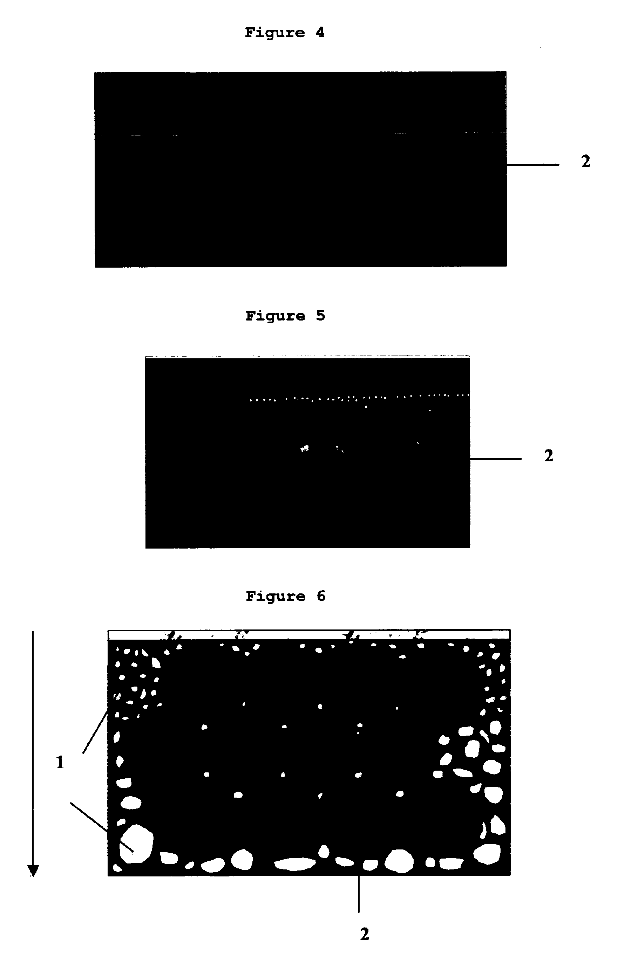

As detailed above the porosity gradient of the polymer tissue engineering scaffold (1) is of utmost importance since it defines the pattern of porosity that permits the colonization of the structure by different cell types. Said porosity can be adapted and/or adjusted to the host tissue nature in order to restore as much as possible the properties of said host tissue in the physiological conditions. As shown on figures 5 to 7, the gradient is manufactured from the internal part to the external part of the implant. It can also be manufactured from the external part to the internal part of the implant. Such gradient is able to help the implant colonization and also to prevent the colonization by inflammatory cells, bacteria or fungi.

In the present invention, the terms below are defined as follows:
"**Mechanically stable template**" refers to an object such as an implant that will not be destabilized by any influence of its environment (fluids, chemicals, pressure, pH, etc.). In animals and especially mammals, a mechanically stable template will remain structurally the same under physiological conditions.
"**Tissue**" refers to any aggregate of cells that perform specific functions; a tissue needs not specifically to form a layer. In animals and more specifically mammalians, the fundamental types of tissues are epithelial, nerve, connective, muscle, and vascular tissues. As examples of mammalian tissues we can mention the bone marrow, the connective tissue that consists of cells that make up fibers in the framework supporting other body tissues and the lymphoid tissue that is part of the immune system and helps protect it from bacteria and other foreign elements.
"**Tissue engineering scaffold**" refers to a structure capable of supporting three-dimensional tissue formation. These scaffolds usually serve at least one of the following purposes:
   - allow cells attachment and migration
   - deliver and retain cells and biochemical factors
   - enable diffusion of vital cell nutrients and expressed products
   - exert certain mechanical and biological influences to modify the behaviour of the cell phase
      Within the meaning of the present invention such structures consist in a natural or a synthetic material selected from:
      - synthetic polymeric materials such as poly(lactic acid) or polylactide also called PLA, poly-L-lactic acid also called PLLA, poly(D,L-lactic acid) or PDLLA, poly (glycolic acid) or PGA, poly(lactic-co-glycolic acid) or PLGA, polycaprolactone or PCL, poly(3-hydroxybutyrate-*co*-3-hydroxyvalerate) or PHBV, and their derivatives, and the co-polymers containing them;
      - natural polymers such as gelatin, alginate, chitosan, collagen, etc. and their derivatives;
      - inorganic material such as metals and ceramics.
"**Porosity**" refers to the condition of a solid material having pores or open spaces.
"**Porous implant**" refers to a solid element having a molecular arrangement of particles which is permeable, thus allowing the passing of air, liquids, tissue or cells into the internal and on the external surfaces, and from the internal surface to the external surface, of said element.
"**Open porosity**" refers to a porosity that is not limited in space, i.e. air or a liquid as examples can go through the entire porous object thanks to the communication between the pores in all the directions without any restriction.
"**Gradient of porosity**" or "**porosity gradient**" refers to a variation within the porous element in the average pore diameter, or pore distribution, concentration of components, or a combination thereof.

According to a particular embodiment of the present invention, the polymer tissue engineering scaffold consists in a biocompatible and biodegradable polymer. In fact, this tissue engineering scaffold is aimed at acting as a temporary support for the cells to attach, move, proliferate and differentiate. After having played that role, the scaffold should degrade, leaving behind it a healthy and functional regenerated tissue. Such biocompatible and biodegradable polymers are selected from: polylactic acid (PLA), poly-L-lactide (PLLA), poly(D,L-lactide) (PDLLA), polyglcolic acid (PGA), poly(lactic-*co-*glycolic acid) (PLGA), polyorthoesters (POE), polycapralactone (PCL), polydiaxonone (PDO), polycaprolactone (PCL), poly-3-hydroxybutyrate (P3HB), poly(3-hydroxybutyrate-*co*-3-hydroxyvalerate) (PHBV), polyvynal fumarate, and combinations thereof, and their derivatives, and the co-polymers containing them. In yet a preferred embodiment, the biocompatible and biodegradable polymer is a synthetic polymer selected from polylactic acid (PLA), poly-L-lactide (PLLA), poly(lactic-*co-*glycolic acid) (PLGA), polycaprolactone (PCL), poly(3-hydroxybutyrate-*co*-3-hydroxyvalerate) (PHBV), combinations thereof, their derivatives, and the co-polymers containing them. In a particular embodiment, the invention relates to a hybrid microporous implant according to the present invention where the biocompatible and biodegradable polymer is selected from poly-L-lactide also called PLLA, and poly(D,L-lactide) also called PDLLA, with a molecular weight comprised between 75000 and 120000.

In yet another particular embodiment, the biocompatible and biodegradable polymer is a natural polymer selected from alginate, chitosan, gelatin, collagen, combinations thereof, and derivatives thereof.

The hybrid implant according to the invention can be manufactured according to the phase separation principle applied on a mechanically stable template. The mechanically stable template is biocompatible, has an open porosity and is not biodegradable. It can be made of any material displaying those characteristics. In a preferred embodiment, the mechanically stable template is made of microporous titanium. The present invention relates also to a method for obtaining a hybrid microporous implant comprising a polymer tissue engineering scaffold which porosity can be controlled combined with a mechanically stable template with open porosity, comprising the following stages:
a) selecting a mould corresponding to the shape of the mechanically stable template;
b) mixing the selected polymer in an appropriate solvent;
c) heating the polymer solution to induce phase separation;
d) placing the mechanically stable template into the mold;
e) seeping the polymer solution into the template in an amount which has been calculated according to the porosity of said template;
f) incubating the hybrid template/polymer to induce gel formation at room temperature;
g) freezing the hybrid;
h) placing the mould containing the frozen hybrid template/polymer in another solvent solution to induce replacement of the solvents;
i) extracting the hybrid template/polymer from the mould and have it dry.

The method of manufacturing a hybrid implant of the invention can be applied to implants with planar structures but also to implants that are cylindrical, oval, square, cubic, star shaped, plug-like, etc. The moulds are corresponding to the template shape and also to the final hybrid implant to manufacture. Consequently the moulds can have various shapes among which cylindrical, oval, square, cubic, star shaped, plug-like, etc.

In a preferred embodiment, the method for obtaining a hybrid implant according to the invention

A method for obtaining a hybrid microporous implant according to the invention comprises the following stages:
a) selecting a mould corresponding to the shape of the mechanically stable template;
b) mixing the selected polymer in an appropriate solvent;
c) heating the polymer solution up to 60°C to induce phase separation;
d) placing the mechanically stable template into the mould;
e) seeping the polymer solution into the template in an amount which has been calculated according to the porosity of said template;
f) incubating the hybrid template/polymer to induce gel formation at room temperature;
g) freezing the hybrid at -80°C;
h) placing the mould containing the frozen hybrid template/polymer in another solvent solution at -20°C to induce replacement of the solvents;
i) extracting the hybrid template/polymer from the mould and have it air-dried.

In the implementation of the method for obtaining a hybrid microporous implant according to the invention, it is particularly suitable that the mould consists of different parts that can be detached separately from each other.

A particularly preferred method according to the present invention involves the use of the polymer poly-L-lactide (PLLA), of the first solvent dioxane and of the second solvent ethanol.

In a particular embodiment the methods described above further compris the step of coating the less porous surface of the hybrid implant with a thin film layer with nanoporosity following the same procedure as b), c) and e).

The principle of the manufacture of a tissue engineering scaffold with porosity is known by one skilled in the art. As prior art we can mention the method of fabrication of porous poly(l-lactide) (PLLA) scaffolds for tissue engineering using liquid-liquid phase separation and freeze extraction (Budyanto L. et al., Journal of Materials Science: Materials in Medicine 2009;20:105-11), the method of preparation of porous scaffolds by using freeze-extraction and freeze-gelation methods (Ho M.-H. et al., Biomaterials, 2004, 25:129-38), but also the self-assembly of peptide amphiphile nanofibers within porous titanium to manufacture a hybrid bone implant (Sargeant T.D. et al., Biomaterials, 2008;29:161-71).

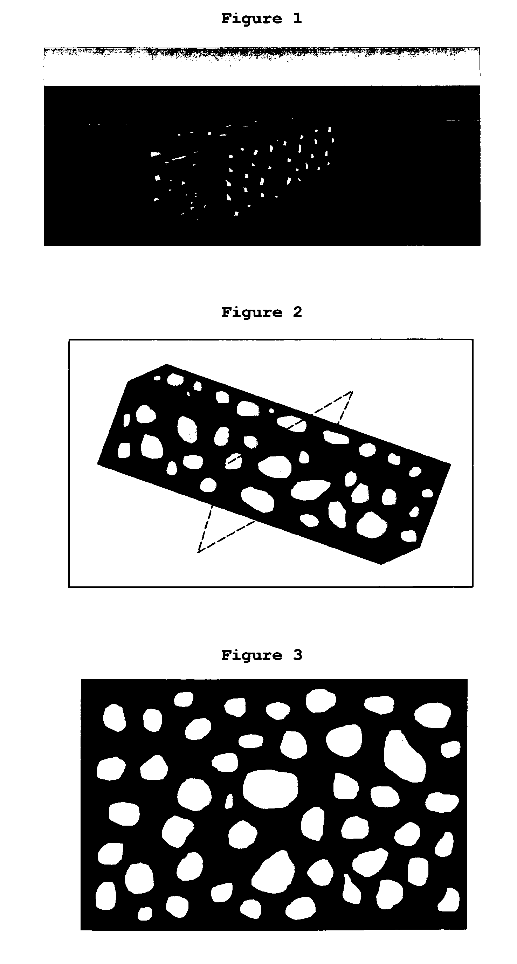

Figures 1 to 3 show a microporous implant according to the prior art. Said implant is the one described in EP0856299 (Debry et al.) or EP1940480 (Debry et al.) and can be obtained by the method described in EP1940480 (Debry et al.). As stated above, such implants do fulfil the mechanical requirements of an implant for in-vivo implantation but they do not fulfil all the requirements for tissue functioning restoration. The implants are shown as an example of an implant with open porosity.

Figures 4 to 6 show a standard microporous implant according to the present invention. The figure 4 shows a mechanically stable template (2) with open porosity. The figures 5 and 6 show a cross-section of an implant according to the invention.

The mechanically stable template (2) is shown with ordered beads in contact with each other and the polymer tissue engineering scaffold (1) is shown with elements with regularly growing sizes thus forming a gradient.

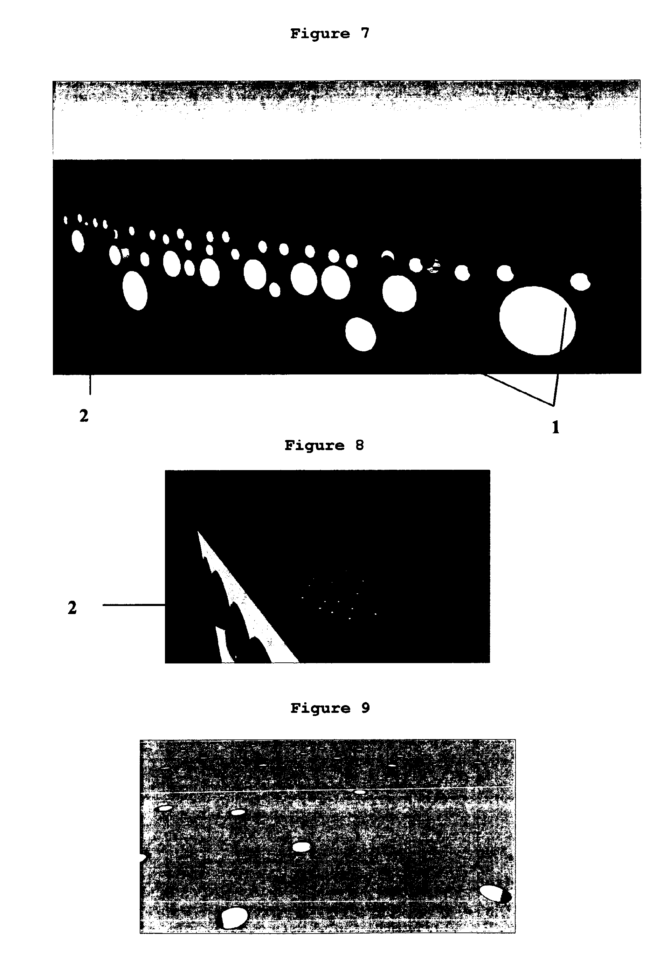

Figures 7 to 9 show the porosity gradient according to the invention. Figure 7 shows an implant surface with the mechanically stable template (2) shown with ordered beads and the polymer tissue engineering scaffold (1) shown with elements with regularly growing sizes. Figure 8 shows the nanoporous surface of an implant according to the invention.

Figure 9 shows a detailed view of the nanoporous surface of the implant of figure 8.

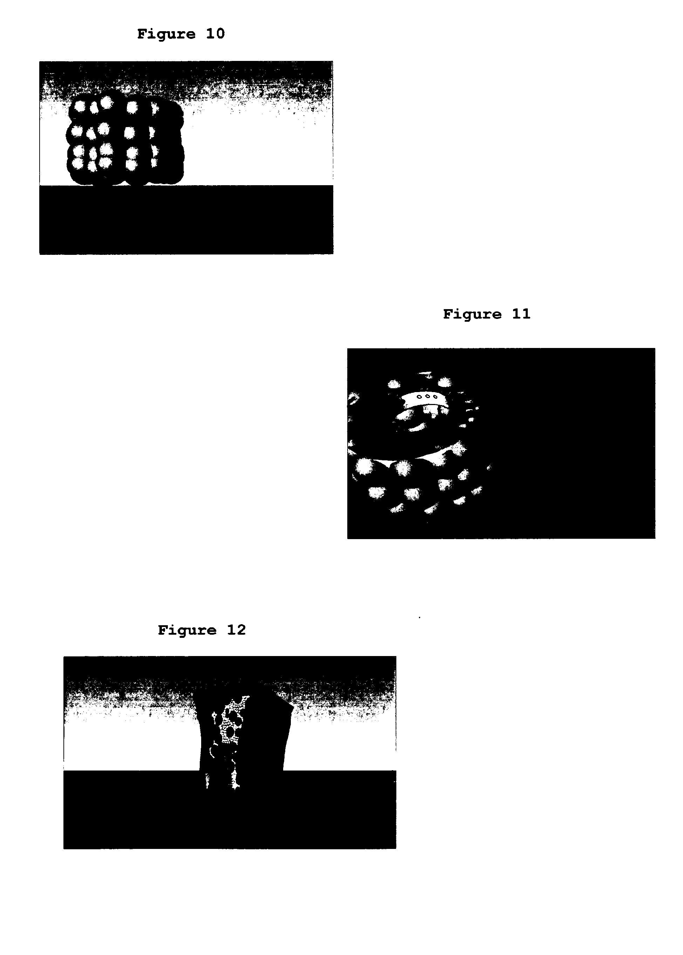

Figures 10 to 12 show various biomedical applications of the present invention. Figure 10 shows a tracheal implant with open porosity according to the prior art. Such an implant is to be positioned in a fistula within the tracheo-esophageal wall of a patient in need thereof. Figure 11 shows a cross-section of a porous tracheal implant according to the invention with open porosity and a polymer tissue engineering scaffold. Figure 12 shows a cross-section of a tooth implant according to the invention.

### Examples

### 1. Support and/or replacement of a trachea

The hybrid implant of the invention is aiming to replace a dysfunctional trachea. The target area is composed of several cell types with different needs and the hybrid implant needs to accommodate these needs in order to be well integrated within the host tissue.

The trachea consists in a series of cartilage rings held together by ligaments that form a lumen that is covered with a layer of pseudo stratified ciliary respiratory epithelial cells. This epithelium lies on a thin layer of basement membrane, which is totally different from the extracellular matrix of the cartilage rings. The cartilage rings provides the mechanical stability and prevents the collapse of the airway, so a robust microporous implant in itself would be sufficient for support and/or replacement of the cartilage function. But the above mentioned thin layer necessary for the formation of a continuous epithelium would be absent.

A tracheal implant according to the present invention fulfils all the requirements of a tissue replacement: the mechanical support is ensured by the stable template and the tissue function restoration is ensured by the gradient of porosity in addition to the microporosity of the stable template.

### A. Template

The mechanically stable template (2) according to the invention is a microporous titanium implant manufactured following the procedure described in EP1940480 (Debry et al.).

### B. Tissue engineering scaffold

The template (2) described in A part above is filled with poly-L-lactide (PLLA) following the method described below in part C. PLLA is a well known biocompatible polymer that has a degradation time of 2 to 12 months within a human organism (Yang et al., 2001).

### C. Method

The microporous titanium implant is placed into a Teflon® mould that is composed of detachable parts in such a way that different parts can be detached separately to control the liquid flow during solvent exchange process. The external surface of the titanium implant is in contact with the mould. PLLA, a biocompatible and biodegradable polymer is dissolved up in a tertiary dioxane/water mixture until a homogenous solution is achieved. The solution is then heated at 60°C to induce better phase separation. After placement of the implant into the mould, the polymer solution is seeped into the implant body in an amount that has been calculated from the porosity of the implant. For an implant having a cylindrical disk form, with a diameter of 11 mm and a thickness of 1,6 mm one will use a volume of polymer solution equal to 152 µl. When the pores are completely filled with the polymer solution, the resulting hybrid body is incubated at room temperature to induce gel formation. The hybrid body is then placed into a freezer at -80°C overnight. The frozen hybrid body is then placed into a 80% cold ethanol solution at -20°C to induce replacement of the dioxane by ethanol. During this process, interconnected open pores are formed. Since one side of the body is in contact with the Teflon® mould, the infusion of the ethanol can only happen from one direction and since infusion changes the structure of the body, the smaller pores are formed on the side of the implant that faces the Teflon® mould. The hybrid implant is then extracted from the mould and air-dried. The pore gradient between the internal and the external surfaces of the hybrid implant starts with big micropores at the internal surface and ends up with small micropores at the external surface. Said pore gradient can be controlled by variations in the polymer concentration, the temperature of heating, the freezing incubation time, the rate of freezing, the mode of freezing and the drying mode.

### 2. Optimization of the tracheal implant

To further decrease the external surface porosity of an implant according to example 1, the phase separation technique can be used in a secondary step where a few microliters of the same polymer as in 1.B. is dissolved in a more volatile solvent such as chloroform and placed homogenously onto the external surface of the implant. The hybrid implant is then immediately immersed into a cold ethanol solution to induce small pore formation in this thin layer. After removal from ethanol solution and air drying, the hybrid implant is covered on its internal surface with a nanoporous layer.

A similar surface coating of the implant with a nanoporous surface coating can also be achieved by developing polyelectrolyte multilayer formation, which was demonstrated with collagen/alginate PEM coatings for the current system.

Although the present invention has been described with reference to particular embodiments, it is to be understood that these embodiments are merely illustrative of the principles and applications of the present invention. It is therefore to be understood that numerous modifications may be made to the described embodiments and that other arrangements may be devised without departing from the spirit and scope of the present invention as defined by the following claims.

## Claims

1. A hybrid microporous implant for the support and/or the replacement of tissue, **characterized in that** it comprises a polymer tissue engineering scaffold (1) which porosity can be controlled, combined with a mechanically stable template (2) with open porosity.

2. A hybrid microporous implant for the support and/or the replacement of tissue according to claim 1, **characterized in that** it comprises a polymer tissue engineering scaffold (1) with a gradient of porosity combined with a mechanically stable template (2) with open porosity.

3. A hybrid microporous implant according to any of claims claim 1 or 2, where the polymer tissue engineering scaffold (1) consists in a biocompatible and biodegradable polymer.

4. A hybrid microporous implant according to claim 3 where the biocompatible and biodegradable polymer is a synthetic polymer selected from polylactic acid (PLA), poly-L-lactide (PLLA), poly(lactic-*co*-glycolic acid) (PLGA), polycaprolactone (PCL), poly(3-hydroxybutyrate-*co*-3-hydroxyvalerate) (PHBV), combinations thereof, their derivatives, and the co-polymers containing them.

5. A hybrid microporous implant according to claim 3 where the biocompatible and biodegradable polymer is a natural polymer selected from alginate, chitosan, gelatin, collagen, combinations thereof, and derivatives thereof.

6. A hybrid microporous implant according to any of the claims 1 to 5 where the mechanically stable template (2) with open porosity is made of microporous titanium.

7. A method for obtaining a hybrid microporous implant comprising a polymer tissue engineering scaffold (1) which porosity can be controlled combined with a mechanically stable template (2) with open porosity, **characterized in that** it comprises the following stages:
a) selecting a mould corresponding to the shape of the mechanically stable template;
b) mixing the selected polymer in an appropriate solvent;
c) heating the polymer solution to induce phase separation;
d) placing the mechanically stable template into the mould;
e) seeping the polymer solution into the template in an amount which has been calculated according to the porosity of said template;
f) incubating the hybrid template/polymer to induce gel formation at room temperature;
g) freezing the hybrid;
h) placing the mould containing the frozen hybrid template/polymer in another solvent solution to induce replacement of the solvents;
i) extracting the hybrid template/polymer from the mould and have it dry.

8. A method for obtaining a hybrid microporous implant according to claim 7, **characterized in that** it comprises the following stages:
a) selecting a mould corresponding to the shape of the mechanically stable template;
b) mixing the selected polymer in an appropriate solvent;
c) heating the polymer solution up to 60°C to induce phase separation;
d) placing the mechanically stable template into the mould;
e) seeping the polymer solution into the template in an amount which has been calculated according to the porosity of said template;
f) incubating the hybrid template/polymer to induce gel formation at room temperature;
g) freezing the hybrid at -80°C;
h) placing the mould containing the frozen hybrid template/polymer in another solvent solution at -20°C to induce replacement of the solvents;
i) extracting the hybrid template/polymer from the mould and have it air-dried.

9. A method for obtaining a hybrid microporous implant according to any of claims 7 or 8, **characterized in that** the mould consists of different parts that can be detached separately from each other.

10. A method for obtaining a hybrid microporous implant according to any of claims 7 to 9, **characterized in that** the mechanically stable template with open porosity is made of microporous titanium.

11. A method for obtaining a hybrid microporous implant according to any of claims 7 to 10, **characterized in that** the polymer is poly-L-lactide (PLLA), the first solvent is dioxane and the second solvent is ethanol.

12. A method for obtaining a hybrid microporous implant according to claim 7, **characterized in that** it further comprises the step of:
j) coating the less porous surface of the hybrid implant with a thin film layer with nanoporosity following the same procedure as b), c) and e).
